# EUROPEAN PATENT APPLICATION

(11) **EP 1 553 180 A1**
(43) Date of publication of application: **13.07.2005**
(21) Application number: 03788123.2
(22) Date of filing: 15.08.2003
(51) Int. Cl.: C12N 15/09, C12N 5/16, C12Q 1/02, A01K 67/027

(54) **NONHUMAN MODEL ANIMAL NONRESPONSIVE TO ENDOTOXIN AND LIPOPROTEIN OR LIPOPEPTIDE**

(30) Priority: 16.08.2002 JP 2002237562
(71) Applicant: Japan Science and Technology Agency, Kawaguchi-shi, Saitama 332-0012 (JP)
(72) Inventor: AKIRA, Shizuo, Takatsuki-shi, Osaka 569-0036 (JP)
(74) Representative: van Westenbrugge, Andries
(86) International application number: PCT/JP2003/010375
(87) International publication number: WO 2004/016082

(57) **Abstract**

The present invention is to provide a non-human animal model non-responsive to bacterial components such as lipoproteins/lipopeptides, peptidoglycans and endotoxins, being useful to elucidate the function of TIRAP being an adaptor protein of TLR family; a substance promoting or suppressing the response to bacterial components using these non-human animal models non-responsive to bacterial components; and a method for screening these promoting or suppressing substances, or the like. TIRAP^{-/-} mice wherein the function of the TIRAP gene is deleted on its chromosome, are generated, and it is confirmed that the responsiveness to ligands recognized by TLR2 such as lipoproteins/lipopeptides and to ligands recognized by TLR4 such as endotoxins is specifically impaired in the TIRAP^{-/-} mice.

SEQUENCE LISTING

<110> Japan Science and Technology Corporation

<120> non-human animal model irresponsive to endotoxin and lipoprotein·
lipopeptide

<130> B02-03PCT

<140>
<141>

<150> JP P2002-237562
<151> 2002-08-16

<160> 2

<170> PatentIn Ver. 2.1

<210> 1
<211> 2277
<212> DNA
<213> Mus musculus

<220>
<221> CDS
<222> (405)..(1127)

<400> 1

cagcgtggag ccgcgtagac tttaagaaac aatatcaacc agcataccga aacacagatc 60

ccgagtccta ccaagccact tttcacaggc tttcttgtgt aacctgcatc cagaacagta 120

agtctatatg gagacaccaa gacctaggaa gactgttgaa ggccatggtc agcaggaggt 180

gcaaacccat gcaatctacc tggaatcggc tgtcttcacc attttatgac tttgtttgcc 240

tagctgacgg atctcaaatg ccctcctggc caggtgaagc ggagaacaat cgctctacca 300

agttcatacc ttgatggctg atccatgaag ttcagactgg atctcttctc tcccgccgtt 360

cccccaatgc ctgctctttc atggttagtt tggaggcctg cact atg gct tca tcc 416
                                                 Met Ala Ser Ser
                                                  1

tcc tcc gtc cca gcc tcc tcc act ccg tcc aag aag cct cga gac aag 464
Ser Ser Val Pro Ala Ser Ser Thr Pro Ser Lys Lys Pro Arg Asp Lys
 5                  10                  15                  20

ata gct gac tgg ttc agg cag gct ctg ttg aag aag ccc aag aag atg 512
Ile Ala Asp Trp Phe Arg Gln Ala Leu Leu Lys Lys Pro Lys Lys Met
                25                  30                  35

ccg atc tcc cag gaa agc cac ctc tat gat ggt tca cag aca gcc aca 560

Pro Ile Ser Gln Glu Ser His Leu Tyr Asp Gly Ser Gln Thr Ala Thr
            40                  45                  50
cag gat ggt ctc tca ccc tcg agc tgc agc tca ccc ccg agt cac agt 608
Gln Asp Gly Leu Ser Pro Ser Ser Cys Ser Ser Pro Pro Ser His Ser
        55                  60                  65
tca ccc gag agc cgt agc tca ccc tcg agc tgc agt tca gga atg tca 656
Ser Pro Glu Ser Arg Ser Ser Pro Ser Ser Cys Ser Ser Gly Met Ser
    70                  75                  80
cct acc tcg cca cca aca cac gtg gac agc agc agc agc agc agt ggc 704
Pro Thr Ser Pro Pro Thr His Val Asp Ser Ser Ser Ser Ser Ser Gly
85                  90                  95                  100
cgc tgg agc aaa gac tac gat gtc tgc gtg tgc cac agt gag gag gac 752
Arg Trp Ser Lys Asp Tyr Asp Val Cys Val Cys His Ser Glu Glu Asp
                105                 110                 115
ttg gag gcg gcc cag gag ctg gtc tcc tac ttg gag ggt agc cag gcc 800
Leu Glu Ala Ala Gln Glu Leu Val Ser Tyr Leu Glu Gly Ser Gln Ala
            120                 125                 130
agt cta cgc tgc ttc ctg cag ctt cgg gat gca gcc ccg ggt ggc gcc 848
Ser Leu Arg Cys Phe Leu Gln Leu Arg Asp Ala Ala Pro Gly Gly Ala
        135                 140                 145

att gtt tcg gag cta tgc cag gca ctg agt cgt agt cac tgc cgt gtg 896
Ile Val Ser Glu Leu Cys Gln Ala Leu Ser Arg Ser His Cys Arg Val
    150                 155                 160

ctg ctc atc act cca ggc ttc ctt cgg gac ccc tgg tgc aag tac cag 944
Leu Leu Ile Thr Pro Gly Phe Leu Arg Asp Pro Trp Cys Lys Tyr Gln
165                 170                 175                 180

atg ctg cag gcc ctg acg gag gcc ccg gcg tcg gag ggt tgc acc ata 992
Met Leu Gln Ala Leu Thr Glu Ala Pro Ala Ser Glu Gly Cys Thr Ile
                185                 190                 195

ccc ctg ctg tcc ggc ctg tcc aga gcc gcc tac ccg ccg gaa ctc cga 1040
Pro Leu Leu Ser Gly Leu Ser Arg Ala Ala Tyr Pro Pro Glu Leu Arg
            200                 205                 210

ttc atg tac tat gtg gat ggc aga ggc aag gac gga ggc ttt tac caa 1088
Phe Met Tyr Tyr Val Asp Gly Arg Gly Lys Asp Gly Gly Phe Tyr Gln
        215                 220                 225

gtc aag gag gct gtt ata cac tat ctg gag aca cta agc tgacacttgg 1137
Val Lys Glu Ala Val Ile His Tyr Leu Glu Thr Leu Ser
    230                 235                 240

gctttcataa gaaaagctgg gaatagctca cagcagtcat taatccagta tatacagcag 1197

agccagagcc agctgcaccc tgtttatgac acaggatcag atcgtcacca gcttccatta 1257

tcgtgggccc acaagatggc caggctgaag atgggaacca cccccgcaag gagaccagga 1317

agaagatggg gactccccaa gaagggcagg aggaagccag ggactccccc aggaagacaa 1377

cgaggaagtc agaaattcga gttgtcaagg gactgtgcta tcgggaaggt cagcagtgtc 1437

ccctctgcgc tgaactggat aggaagctga tacatggctg tcttcttcct ctggcatcag 1497

gcacggggga aatggatacc actcctcaag aggggtgaac ccgattgatg agccagaagt 1557

tgcctaggga agctgagaag aggtggcaag tatccagtcc ttcacacaca cacacactac 1617

tgaaactctt ccccagagat gtcaactcac ctcacaaaac tggaagacac gttcatcccc 1677

atactgaatt tcaccttttg aaaaaaaatg tcattaaaaa tgcaccccga ggggctgggg 1737

agacagtggt tcagcggtta agaactcttg ttgcttttcc agaggatcca ggttcaattc 1797

ccaggatcca catggtgact cccaacctta tctataactc tgcttccagg ggatctgatg 1857

tcttctctga ccaagacctc cttgagcacc aggtacacac gtgaacacct ccatagatgc 1917

aggcaaaaca atcatacaca taaagaaatc ttccttttta catcagaagt gcaaacctag 1977

gactctagat ggagtccgtg gtaggcttgc ttagcatacc tgagggcttg gattctacct 2037

ccagcaccac caaaacaaag gagaaaacac aaacctggca gctgctctgt atccagaaac 2097

aggtaagaag aaggaaagcc agggtgagcc aggcggtggt ggcacacgcc tttaatccca 2157

gcactcggga ggcattggca ggcggatttc tgagttcgag gccagcctgg tctacagagt 2217

tcccggacag ccagggctac acagagaaac cctgtctcga aaaaccaaaa aaaaaaaaaa 2277

<210> 2
<211> 241
<212> PRT
<213> Mus musculus

<400> 2
Met Ala Ser Ser Ser Ser Val Pro Ala Ser Ser Thr Pro Ser Lys Lys
1               5                   10                  15

Pro Arg Asp Lys Ile Ala Asp Trp Phe Arg Gln Ala Leu Leu Lys Lys
            20                  25                  30

Pro Lys Lys Met Pro Ile Ser Gln Glu Ser His Leu Tyr Asp Gly Ser
        35                  40                  45

Gln Thr Ala Thr Gln Asp Gly Leu Ser Pro Ser Ser Cys Ser Ser Pro
    50                  55                  60

Pro Ser His Ser Ser Pro Glu Ser Arg Ser Ser Pro Ser Ser Cys Ser
65                  70                  75                  80

Ser Gly Met Ser Pro Thr Ser Pro Pro Thr His Val Asp Ser Ser Ser
                85                  90                  95

Ser Ser Ser Gly Arg Trp Ser Lys Asp Tyr Asp Val Cys Val Cys His
            100                 105                 110

Ser Glu Glu Asp Leu Glu Ala Ala Gln Glu Leu Val Ser Tyr Leu Glu
        115                 120                 125

Gly Ser Gln Ala Ser Leu Arg Cys Phe Leu Gln Leu Arg Asp Ala Ala
    130                 135                 140

Pro Gly Gly Ala Ile Val Ser Glu Leu Cys Gln Ala Leu Ser Arg Ser
145                 150                 155                 160

His Cys Arg Val Leu Leu Ile Thr Pro Gly Phe Leu Arg Asp Pro Trp
                165                 170                 175

Cys Lys Tyr Gln Met Leu Gln Ala Leu Thr Glu Ala Pro Ala Ser Glu
            180                 185                 190

Gly Cys Thr Ile Pro Leu Leu Ser Gly Leu Ser Arg Ala Ala Tyr Pro
        195                 200                 205

Pro Glu Leu Arg Phe Met Tyr Tyr Val Asp Gly Arg Gly Lys Asp Gly
    210                 215                 220

Gly Phe Tyr Gln Val Lys Glu Ala Val Ile His Tyr Leu Glu Thr Leu
225                 230                 235                 240

Ser

## Description

### Technical Field

The present invention relates to a non-human animal model wherein the function of TIRAP gene mediating signaling of Toll-like receptors (TLR) is deleted; especially a TIRAP- knockout mouse non-responsive to ligands recognized by TLR2 and TLR4 such as endotoxins and lipoproteins/lipopeptides (hereinafter referred to as "TLR2 and TLR4 ligands"); or a method for screening substances promoting or suppressing response to TLR2 and TLR4 ligands by using these, or the like.

### Background Art

Toll genes are associated with the determination of the dorsoventral axis in the embryogenesis of Drosophilia (Cell 52, 269-279, 1988; Annu. Rev. Cell Dev. Biol. 12, 393-416, 1996), and with innate immunity detecting invading pathogens in the adult body (Nature 406, 782, 2000; Nat. Immunol. 2, 675, 2001; Annu. Rev. Immunol. 20, 197, 2002). The Toll is a type I-transmembrane receptor having a Leucine-rich repeat (LRR) in the extracellular domain, and among cytokines being the signaling substance between cells which play an important role in the immunoreaction and response during an infection, hematogenesis, viral infection and impairment of tumor cell, cytokine transmitting signals between lymphocytes is called Interleukin (hereinafter referred to as "IL"). It has been clarified that the intracytoplasmic domain of the type I-transmembrane receptor is highly homologous with the intracytoplasmic domain of the mammalian IL-1 receptor (IL-1R) (Nature 351, 355-356, 1991; Annu. Rev. Cell Dev. Biol. 12, 393-416, 1996; J. Leukoc. Biol. 63, 650-657, 1998).

Recently, a mammal homologue of Toll has been identified (Nature 388, 394-397, 1997; Proc. Natl. Acad. Sci. USA95, 588-593, 1998; Blood 91, 4020-4027, 1998; Gene 231, 59-65, 1999) and 10 members of human TLR family such as TLR2 and TLR4 have been reported so far. The role of the TLR family is to recognize discrete pathogen-associated molecular patterns (PAMPs) as pattern recognition receptor (PRR) recognizing common bacterial structure, to trigger the activation of similar intracellular signaling pathway leading to the nuclear translocation of a transcription factor, NF-κB. The signaling pathway ultimately culminates in the production of inflammatory cytokines to evoke host defense responses and further evoke host defense responses to acquired immunity. Moreover, various TLR ligands are reported recently.

TLR2 recognizes a variety of bacterial components, such as peptidoglycan (PGN), bacterial tri-acylated lipoproteins, mycoplasmal di-acylated lipoproteins, and GPI anchor of Trypanosoma cruzi (Science 285, 732, 1999; Science 285, 736, 1999; J. Biol. Chem. 274, 33419, 1999; Immunity 11, 443, 1999; J. Immunol. 164. 554, 2000; Nature 401, 811, 1999; J. Immunol. 167, 416, 2001). TLR4 is essential for responses to lipopolysaccharide (hereinafter LPS), a glycolipid specific to Gram-negative bacteria cell wall. TLR5 is reported to recognize flagellin, a protein component of bacterial flagella. Furthermore, TLRs also detect nucleotides specific to pathogens and nucleotide analogues. TLR 3, TLR 7, and TLR 9 participate in the recognition of viral double-stranded RNA, imidazoquinolines and DNA with bacterial unmethylated CpG motif (5'-Pu-Pu-CpG-Pyr-Pyr-3') (CpG DNA), respectively (Nature 406, 782, 2000; Nat. Immunol. 2, 675, 2001; Annu. Rev. Immunol. 20, 197, 2002; Nat. Immunol. 3, 196, 2002).

In a signal pathway via TLRs, signaling originates from the Toll/interleukin-1 receptor (TIR) domain, in which a signaling pathway from TLR4 is well known (Hoffman, J.A. et al.: Science, 284: 1313-1318, 1999). By the activation of TLR4, Serine/threonine kinase IRAK is activated via MyD88, an adaptor molecule, degradation of IκB being an inhibitor of a transcription factor NF-κB, is induced, ultimately NF-κB translocates to the nuclear and the transcription starts. This signaling pathway from TLR is evolutionarily well conserved. In Drosophilia, Tube corresponds to MyD88, Pelle to IL-1R-associated kinase (hereinafter referred to as IRAK), Cactus to IκB, and Dif or Dorsal to NF-κB, respectively.

MyD88 is an intracytoplasmic protein composed of IL-1R homologous domain and death domain. MyD88 gene is known to be originally isolated as a myeloid cell differentiation primary response gene that is rapidly induced upon IL-6-stimulated differentiation of M1 myeloleukaemic cells into macrophages. MyD88 is involved in IL-1R or IL-18R signals and has a critical role in immune mechanism (Adachi, O. et al.: Immunity, 9: 143-150, 1998). Therefore, it is reported that MyD88 gene-deficient (MyD88^{-/-}) mice are susceptible to infection with bacteria, and are more susceptible especially to Gram-positive bacterial infections than TLR2 gene-deficient (TLR2^{-/-}) mice (Takeuchi, O. et al.: J Immunol., 165: 5392-96, 2000).

Furthermore, MyD88^{-/-} mice show no inflammatory response to most of the microbial components that are recognized by TLRs (Akira et al.: Nature Immunol. 2, 675-680, 2001; Annu. Rev. Immunol. 20: 197-216 2002). Although several LPS responses including induction of IFN-inducible genes and maturation of dendritic cells have been shown in those mice, production of inflammatory cytokines is completely abolished. Moreover, in MyD88-deficient macrophages, production of inflammatory cytokines not just to LPS but also to cell wall components of Gram-positive bacteria, peptidoglycan (PGN), bacterial lipoprotein (BLP), DNA with bacterial unmethylated CpG motif (CpG DNA), and the like is not recognized (Hacker, H. et al.: J. Exp. Med., 192: 595-600, 2000). However, in the case of TLR4-mediated signaling pathway, LPS-induced responses are observed in MyD88^{-/-} mice, indicating the presence of the MyD88-independent signaling pathway (Akira et al.: Nature Immunol. 2, 675-680, 2001; Medzhitov Nature Rev. Immunol. 1: 135-145, 2001). It has been reported that the MyD88-independent pathway contributes to LPS-induced expression of IFN-inducible genes and maturation of dendritic cells (DC) (Kawai et al.: J.Immunol. 167: 5887-94, 2001; Kaisho et al.: J. Immunol. 166: 5688-94, 2001). In vitro studies have indicated that an adaptor molecule termed TIRAP is involved in LPS-induced activation of the MyD88-independent signaling pathway (Horng et al.: Nature Immunol. 2, 835-841, 2001; Toschakov et al.: Nature Immunol. 3, 392-98, 2002).

TIRAP gene is identified as an adaptor that has the TIR domain conserved in TLR family as an intracytoplasmic domain of TLR, and mediates the TLR4 signaling (Horng et al.: Nature Immunol. 2, 835-841, 2001), but physiological function thereof is yet to be clarified. The object of the present invention is to provide a non-human animal model non-responsive to bacterial components such as lipoproteins/lipopeptides being bacterial components belonging to Mycoplasma, Spirochaeta, Escherichia or the like, peptidoglycan being cell wall fractions of Gram-positive bacteria, endotoxins being cell wall fractions of Gram-negative bacteria, or the like, that are useful to elucidate the function of TIRAP being an adaptor protein of TLR family to the signaling by the stimulation of bacterial components in vivo, especially useful in measures for infectious diseases such as endotoxin shock and pneumonia, and in treatment measures in case that the infectious diseases are combined; a substance promoting or suppressing to the bacterial infections using these non-human animal models non-responsive to bacterial components; and a method for screening these promoting or suppressing substances.

The present inventors have made a keen study to overcome the object described above. They substituted two exon domains encoding 3' end potion of the TIRAP gene domain by the homologous recombination with ES cells by using a plasmid vector to a neomycin-resistant gene, introduced HSV-tk gene on 3' end, screened ES cell clones showing double resistance to both G418 and Gancyclovir, injected the ES cell clones into C57BL/6 mice blastocysts to obtain TLR1 knockout mice wherein the function of TIRAP gene is deleted according to the Mendel law through the germline.

These TIRAP^{-/-} mice were healthy and showed no abnormal composition of B and T lymphocytes. Furthermore, in such TIRAP^{-/-} mice, proliferation of splenocytes to LPS and inflammatory cytokine production of peritoneal macrophages were abolished. Similarly to MyD88^{-/-} mice, LPS-induced activation of NF-κB and MAP kinases was delayed, but expression of IFN-inducible genes and maturation of dendritic cells were observed in TIRAP^{-/-} mice. TIRAP^{-/-} mice displayed normal responses to TLR3, TLR7 or TLR9 ligands, however they showed no cytokine production and activation of signaling in response to lipoproteins/lipopeptides and endotoxins, both of which recognized by TLR2 and TLR4. From these findings, the present inventors confirmed that TIRAP is not essential for the activation of LPS-induced MyD88-independent signaling pathway and acts as an adaptor molecule in TLR2 and TLR4 mediated MyD88-dependent signaling pathway. The present invention has been thus completed.

### Disclosure of the Invention

In other words, the present invention relates to a non-human animal model non-responsive to endotoxins and lipoproteins/lipopeptides, wherein the function of the TIRAP gene is deleted in its chromosome and the responsiveness to ligands recognized by TLR2 and TLR4 is specifically impaired ("1"); the non-human animal model non-responsive to endotoxins and lipoproteins/lipopeptides according to "1", wherein the non-human animal model is non-responsive to LPS, PGN, and MALP-2, and responsive to CpG ODN, IL-1, Poly I:C and R-848 ("2"); the non-human animal model non-responsive to endotoxins and lipoproteins/lipopeptides according to "1" or "2", wherein the non-human animal is a mouse ("3"); the non-human animal model non-responsive to endotoxins and lipoproteins/lipopeptides according to "3", wherein the mouse is obtained by a process comprising steps of: obtaining the gene site of TIRAP from screening TIRAP genes from murine genomic library by using a probe from mouse TIRAP gene; constructing a targeting vector by substituting a whole or a part of the gene fragment of the gene site of TIRAP to a plasmid containing a marker gene; linearizing the targeting vector and injecting it into embryonic stem cells; microinjecting the targeted stem cells wherein the function of TIRAP gene is deleted, into the mouse blastocysts to generate chimeric mice; breeding the chimeric mice and wild-type mice to generate heterozygous mice; and intercrossing the heterozygous mice ("4").

Furthermore, the present invention relates to a method for screening substances promoting or suppressing the response to ligands recognized by TLR2 or TLR4, or to the inclusion thereof, wherein the response to ligands recognized by TLR2 or TLR4, or to the inclusion thereof in the immunocytes derived from the non-human animal according to any one of "1" to "4" is measured/estimated, by using the immunocytes, a test substance, and ligands recognized by TLR2 or TLR4, or the inclusion thereof ("5"); the method for screening substances promoting or suppressing the response to ligands recognized by TLR2 or TLR4, or to the inclusion thereof, wherein the response to ligands recognized by TLR2 or TLR4, or to the inclusion thereof in the non-human animal according to any one of "1" to "4" is measured/estimated, by using the non-human animal, a test substance and ligands recognized by TLR2 or TLR4, or the inclusion thereof ("6"); a substance promoting or suppressing the response to ligands recognized by TLR2 or TLR4, or to the inclusion thereof, obtained by the method for screening a substance promoting or suppressing the response to ligands recognized by TLR2 or TLR4, or to the inclusion thereof according to "5" or "6"("7").

### Brief Description of Drawings

Fig. 1 is a figure that shows a genomic locus of the TIRAP-knockout mouse of the present invention, of a wild-type mouse and of a targeting vector.
In Fig. 2, the left figure shows the result of confirmation of homologous recombination in ES cells in the TIRAP-knockout mouse of the present invention and in a wild-type mouse by Southern blot analysis, and the right figure shows the result of analysis using the N terminal fragment and C terminal fragment P of TIRAP gene as a probe in order to confirm the expression of TIRAP mRNA in the knockout mouse of the present invention and in a wild-type mouse, respectively.
Fig. 3 is a figure that shows cDNA sequence and a predicted amino acid sequence of N-terminal portion of the disrupted TIRAP gene in the TIRAP-knockout mouse of the present invention. The number indicates the cDNA position from the start codon and bold letters show intersection of wild-type TIRAP gene product and disrupted one.
Fig. 4 is a figure that shows the result of responsiveness of splenocytes from the TIRAP-knockout mouse of the present invention and wild-type mouse to LPS and CpG ODN respectively.
Fig. 5 is a figure that shows the expression of MHC class II on the surface of splenic B cells from the TIRAP-knockout mouse of the present invention and from a wild-type mouse.
Fig.6 is a figure that shows the results of measurement of the proliferation of splenocytes from the TIRAP-knockout mouse of the present invention and from a wild-type mouse in the presence of IL-1, and of PHA, and in the co-presence of both IL-1 and PHA.
Fig. 7 is a figure that shows the results of measurement of the LPS-induced inflammatory cytokine production of peritoneal macrophages from the TIRAP-knockout mouse of the present invention and from a wild-type mouse.
Fig. 8 is a figure that shows the results of measurement of the R-848- and CpG ODN-induced inflammatory cytokine production of peritoneal macrophages from the TIRAP-knockout mouse of the present invention and from a wild-type mouse.
Fig. 9 is a figure that shows the results of measurement of the MALP-2- and S.aureus PGN-induced inflammatory cytokine production of peritoneal macrophages from the TIRAP-knockout mouse of the present invention and from a wild-type mouse.
Fig. 10 is a figure that shows the result of analysis of Poly I:C-induced expression of IFN-β mRNA of peritoneal macrophages from the TIRAP-knockout mouse of the present invention and from a wild-type mouse.
Fig. 11 is a figure that shows the result of stimulating peritoneal macrophages from the TIRAP-knockout mouse of the present invention and from a wild-type mouse with LPS, MALP-2, or R-848, and visualized.
Fig. 12 is a figure that shows the result of stimulating peritoneal macrophages from the TIRAP-knockout mouse of the present invention and from a wild-type mouse with LPS, MALP-2 or R-848, and analyzing the activation of MAP kinases.
Fig. 13 is a figure that shows the result of stimulating peritoneal macrophages from the TIRAP-knockout mouse of the present invention and from a wild-type mouse with LPS, MALP-2 or R-848, and analyzing the activation of IRAK-1 phosphorylation.
Fig. 14 is a figure that shows the result of stimulating peritoneal macrophages from the TIRAP-knockout mouse of the present invention, from a wild-type mouse and from a MyD88 knockout mouse with LPS, and analyzing the expression of IP-10, GARG-16, and IRG-1.
Fig. 15 is a figure that shows the result of stimulating bone marrow-derived dendritic cells from the TIRAP-knockout mouse of the present invention and from a wild-type mouse with LPS, and analyzing the amount of antigen expressed on the surface of the cells.
Fig. 16 is a figure that shows the result of stimulating peritoneal macrophages from the TIRAP-knockout mouse of the present invention and from a wild-type mouse with LPS, and detecting monomeric and dimeric forms of IRF-3.
Fig. 17 is a figure that shows the result of stimulating peritoneal macrophages from the TIRAP-knockout mouse of the present invention and from a wild-type mouse with LPS, and detecting phospho-PKR by Western blot analysis.
Fig. 18 is a figure that shows the result of stimulating peritoneal macrophages from the TIRAP/MyD88 double-knockout mouse of the present invention and from a wild-type mouse with LPS, and analyzing the expression of IP-10, GARG-16, and IRG-1.
Fig. 19 is a figure that shows the result of stimulating bone marrow-derived dendritic cells from the TIRAP/MyD88 double- knockout mouse of the present invention and from a wild-type mouse with LPS, and analyzing the amount of antigen expressed on the surface of the cells.

### Best Mode of Carrying Out the Invention

As for a non-human animal model non-responsive to endotoxins and lipoproteins/lipopeptides of the present invention, there is no specific limitation as long as it is a non-human animal model, wherein the function of the TIRAP gene is deleted on its chromosome, and both responsiveness to ligands recognized by TLR2 and TLR4 are specifically impaired. The present invention is also directed to a multiple gene deficient non-human animal model wherein the functions of other genes such as the MyD88 gene in addition to the TIRAP gene are deleted on its chromosome. As for ligands recognized by the above-mentioned TLR2, peptidoglycan (PGN) being a skeleton structure of bacterial cell wall wherein relatively short peptide chain binds to repeated polysaccharide of N-acetylglucosamin and N-acetylmuramic acid, bacterial macrophage-activating lipopeptides belonging to Mycoplasma (MALP-2), bacterial tri-acylated lipoproteins, Mycoplasmal diacylated lipoproteins, lipoproteins/lipopeptides being bacterial components belonging to Mycoplasma such as GPI anchor of Trypanosoma cruzi, Spirochete, Escherichia or the like can be exemplified. As for ligands recognized by TLR4, lipopolisaccharide (LPS) also termed as endotoxin mainly existing as outer membrane components of Gram-negative bacteria, lipoteichoic acid (LTA), Mycobacterium tuberculosis lysates, the cell wall fraction of Gram-positive bacteria being cell wall components of Gram-positive bacteria or the like can be exemplified. Moreover, in the present invention, for convenience, ligands recognized by TLR2 or TLR4 as well as carrier carrying these ligands, and bacterial cell itself having these ligands on the surface of the cells are included as ligands recognized by TLR2 and TLR4 of the present invention (hereinafter referred to as "ligands of the present invention").

In the present invention, non-responsiveness means that the responsiveness of the living body or of the cells, tissues or organs composing the living body to the stimulation by ligands of the present invention is reduced or almost deleted. Therefore, in the present invention, a non-human animal model non-responsive to endotoxins and lipoproteins/lipopeptides refers to animals other than human such as mice, rats, and rabbits wherein the responsiveness of the living body or of the cells, tissues or organs composing the living body to the stimulation by ligands of the present invention is reduced or almost deleted. Moreover, as for the stimulation by ligands of the present invention, in vivo stimulation administering endotoxins and lipoproteins/lipopeptides to the living body, or in vitro stimulation contacting endotoxins and lipoproteins/lipopeptides to cells separated from the living body can be exemplified.

As for a non-human animal model non-responsive to endotoxins and lipoproteins/lipopeptides of the present invention, a non-human animal non-responsive to ligands of the present invention, such as bacterial components or the like including lipoproteins/lipopeptides, peptidoglycans, cell wall fractions of Gram-positive bacteria, endotoxins, lipoteichoic acids, and Mycobacterium tuberculosis lysates or the like, but responsive to CpG oligodeoxynucleotides (CpG ODN) being TLR9 ligands, IL-1, phytohemagglutinin (PHA), Poly I:C being TLR3 ligands, and synthetic compound R-848 being a TLR7 ligand can be exemplified. For instance, a non human animal wherein the function of TIRAP gene is deleted on its chromosome can be exemplified.

In the present invention, deletion of the function of TIRAP gene means that a part or a whole of TIRAP gene is deleted on its chromosome, and the function of expressing TIRAP being expressed in wild-type is deleted. As for an animal wherein the function of TIRAP gene is deleted, TIRAP^{-/-} mice as well as rodents such as rats wherein the function of TIRAP gene is deleted can be exemplified. As for a non-human animal wherein the function of TIRAP gene is deleted, TIRAP^{-/-} non-human animal generated upon Mendel's law is preferable from the point of view of being able to obtain wild-types being its littermates at the same time, with which accurate comparative examples can be conducted. The method for generating an animal wherein the function of TIRAP gene is deleted will be explained by taking TIRAP^{-/-} mouse as an example.

TIRAP genes can be amplified from murine genomic library by PCR method or the like, and the obtained-gene fragments can be screened using a probe derived from mouse TIRAP gene, and the screened TIRAP genes are subcloned using plasmid vectors and the like and can be identified by restriction enzyme mapping and DNA sequencing. Next, a whole or a part of the gene encoding TIRAP is replaced with pMC 1 neo-gene cassette and the like, and targeting vectors are prepared by introducing genes such as Diphtheria-toxin A fragment (DT-A) genes or herpes simplex virus thymidine kinase (HSV-tk) genes to 3'end.

The prepared targeting vectors are linearized and introduced into ES cells by electroporation and the like for homologous recombination, and ES cells having performed homologous recombination by antibiotics such as G418 or Gancyclovir (GANC) are selected from the homologous recombinants. Moreover, it is preferable to confirm if the selected ES cells as the desired recombinants by Southern blot method or the like. Clones of the confirmed ES cells are microinjected into mouse blastocysts which are returned to recipient mice, to generate chimeric mice. The chimeric mice are bred to wild-type mice to obtain heterozygous mice (F1 mice: +/-), and by breeding the heterozygotes, TIRAP^{-/-} mice of the present invention can be generated. Moreover, as for a method for confirming whether TIRAP has been deleted in TIRAP^{-/-} mice, a method by isolating RNA from a mouse obtained by the above-mentioned method and examining by Northern blot method or the like, or a method by examining TIRAP expression in the mice can be examined by Western blot method or the like, can be exemplified.

Furthermore, it can be confirmed whether the generated TIRAP^{-/-} mice are non-responsive to ligands of the present invention, by for example contacting lipoproteins/lipopeptides being the bacterial components belonging to Mycoplasma, Spirochaeta, Escherichia and the like to immunocytes of TIRAP^{-/-} mice such as macrophages, mononuclear cells and dendritic cells, in vitro or in vivo, and by measuring the production levels of TNF-α, IL-6, IL-12 and the like in said cells, the proliferative response of splenocytes, the expression level of antigens such as CD 40, CD80, CD86, MHC class II on the surface of splenocytes, or the activation of molecules in the signaling pathway such as NF-κB, JNK, IRAK; by administering LPS being bacterial cell wall components of Gram-negative bacteria to TIRAP-knockout mice by intravenous injection or the like and measuring the biological activity of endotoxins such as pyrexia, shock, reduction of leukocytes or platelets, hemorrhagic necrosis in bone marrow, hypoglycemia, IFN induction, activation of B-lymphocytes (bone marrow-derived immune response cell); and by measuring, for example, TNF induction, proliferation response of splenocytes, or expression of MHC class II antigens on the surface of splenocytes in macrophages and splenocytes of the TIRAP-knockout mice in the presence of bacterial LPS, or peptidoglycan, lipoteichoic acids, Mycobacterium tuberculosis lysates or the like being Gram-positive bacterial components.

A TIRAP^{-/-} mouse of the present invention is non-responsive to lipoproteins/lipopeptides being bacterial components, and has a lower responsiveness to endotoxins than C3H/HeJ mice known to have low-responsiveness to endotoxins to date. Symptoms of shock are not observed at all. Macrophages and splenocytes derived from TIRAP^{-/-} mice are non-responsive not only to endotoxins but also to peptidoglycans, lipoteichoic acids, Mycobacterium tuberculosis lysates and the like being cell wall components of Gram-positive bacteria, while being responsive to IL-4 or IFN-γ. Therefore, knockout mice non-responsive to ligands of the present invention can be useful models to elucidate the effect mechanism of lipoproteins/lipopeptides, endotoxins, peptidoglycans, lipoteichoic acids or the like, or to investigate a treatment strategy for bacterial infections such as endotoxin shock, tuberculosis and pneumonia.

Non-human animal models non-responsive to endotoxins and lipoproteins/lipopeptides of the present invention, or immunocytes such as macrophages, splenocytes, dendritic cells derived from said model animals can be used for screening substances promoting or suppressing the response to TLR2 and TLR4 ligands or the like, besides for elucidating the effect mechanism of TLR2 and TLR4. As for the method for screening substances promoting or suppressing the response to ligands of the present invention, there is no specific limitation as long as it is a method for screening to measure/estimate the response to ligands of the present invention in immunocytes derived from non-human animal models of the present invention, by using the immunocytes, a test substance and ligands of the present invention; or a method for screening to measure/estimate the response to ligands of the present invention in the non-human animal models of the present invention, by using the non-human animal models, a test substance and ligands of the present invention. The embodiment of the method for screening of the present invention will be explained in the following by referring to the examples.

A method for screening comprising the steps to culture immunocytes such as macrophages, splenocytes or dendritic cells obtained from a non-human animal model of the present invention, a test substance and ligands of the present invention together, and measure/estimate the level of cell activity in the immunocytes; a method for screening comprising the steps to administer previously a test substance to a non-human animal model of the present invention, then to culture the immunocytes obtained from the non-human animal in the presence of ligands of the present invention, and to measure/estimate the level of cell activity in the immunocytes; a method for screening comprising the steps to administer previously ligands of the present invention to a non-human animal model of the present invention, then to culture the immunocytes obtained from the non-human animal in the presence of a test substance, and to measure/estimate the level of cell activity in the immunocytes; and a method for screening comprising the steps to administer ligands of the present invention and a test substance concurrently, or one of them in advance of the other, and to measure/estimate the level of cell activity in the immunocytes obtained from the non-human animal model can be exemplified.

As for a method for measuring/estimating level of cell activity in the immunocytes such as macrophages and splenocytes, when immunocytes are macrophages, a method for measuring/estimating the production levels of cytokines such as IL-6, TNF-α, IL-12 in macrophages, or level of DNA binding effect of NF-κB; and when immunocytes are splenocytes, a method for measuring/estimating the level of proliferation of splenocytes or expression level of MHC class II in splenocytes can be exemplified, respectively. Furthermore, when measuring/estimating the level of cell activity in the immunocytes, it is preferable to compare/estimate with the measurement levels of the wild-type non-human animal model of the present invention as control, especially with those of wild-type non-human animals being its littermates, to eliminate individual variety.

A substance promoting or suppressing obtained by the method for screening a substance promoting or suppressing the response to ligands recognized by TLR2 or TLR4 of the present invention, or to the inclusion thereof is useful to elucidate the mechanism in signaling by stimulation of bacterial components in vivo, especially a promoting substance can possibly be used as preventive/treating agents for bacterial infections. When above-mentioned promoting substance wherein preventive/treating effect for bacterial infections can be expected, is used as drugs, various prescribed compounds such as pharmaceutically acceptable normal carrier, bonding agent, stabilizing agent, excipient, diluent, pH buffer agent, disintegrator, solubilizer, dissolving adjuvant, isotonic agent can be added. As for a method for preventing or treating by using these drugs, appropriate dose according to the patient's sex, body weight, symptoms can be administered orally or parenterally. In other words, it can be administered orally in a dosage form used generally, for example in form of powder, granule, capsules, syrup, suspension and the like, or parenterally for example in form of solution, emulsion, suspension and the like by injection, and moreover, it can be administered in nostril in form of spray.

The present invention will be explained in detail in the following by referring to the examples, but the technical scope of the present invention will not be limited to these.

### Example 1 (preparation of TIRAP-knockout mice)

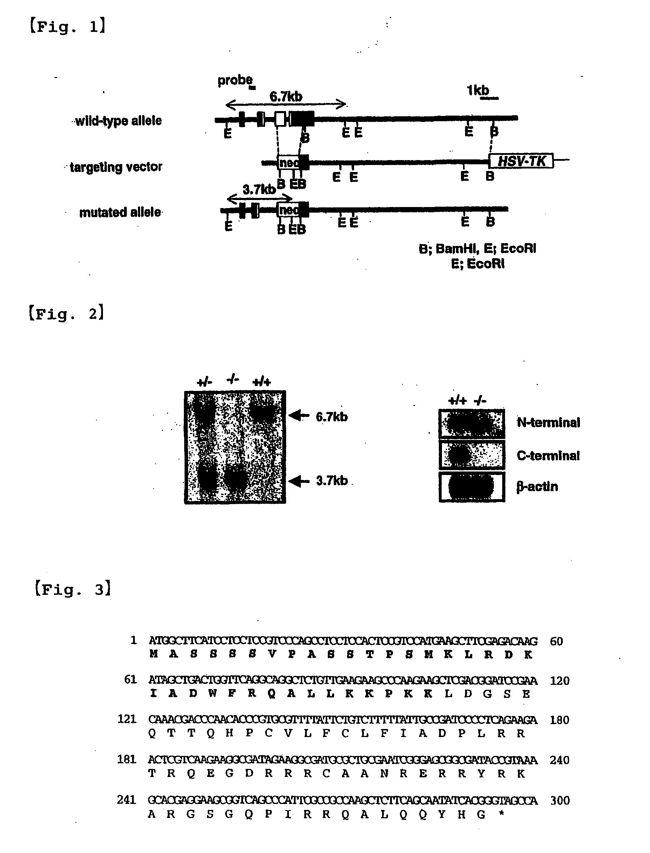

To generate TIRAP-knockout mice by gene targeting, TIRAP genomic DNAs were screened from 129SvJ murine genomic library (Stratagene) using a probe derived from mouse TIRAP gene, further TIRAP genes were isolated by purifying the plasmid containing mouse TIRAP gene, subcloned in pBluescript II SK(+) vector (Stratagene) and confirmed as being TIRAP gene (Seq.ID Nos. 1,2) by restriction enzyme mapping and DNA sequence determination. A targeting vector was constructed by replacing 1.6-kb fragment encoding a part of mouse TIR domain with a neomycin-resistance gene cassette (neo)(Stratagene), and by introducing herpes simplex virus thymidine kinase (HSV-TK) driven by MC1 promoter as a negative selection marker (Fig. 1). The obtained targeting vector was linearized with SalI, introduced into embryonic stem cells (ES cells) by electroporation method. Doubly resistant colonies showing resistance to both G418 and Gancyclovir were selected and screened by PCR. Moreover, homologous recombination of genes in ES cells was confirmed by Southern blot. Homologous recombinants obtained by the screening were microinjected into blastocysts from C57BL/6 female mice, and heterozygous F1 offspring were intercrossed to obtain TIRAP-knockout mice.

To confirm whether the function of TIRAP genes was deleted in homozygous mice (-/-), Southern blot analysis was conducted. It was confirmed by Southern blot method by using gene fragment having digested genomic DNA extracted from mouse murine tail with EcoRI, and the radiolabeled probe derived from mouse TIRAP gene. As a result, Southern blotting gave a single 6.7-kb band for wild-type (+/+), a 2.7-kb band for homozygous (-/-) and both bands for heterozygous mice (+/-) (Fig. 2, left). Furthermore, to confirm whether TIRAP genes were not expressed in homozygous mice(-/-), Northern blot analysis was conducted. Total RNA (10 □g) extracted from embryonic fibroblasts was electrophoresed, transferred to a nylon membrane, and hybridized using the TIRAP N-terminal or C-terminal fragment as a probe. As positive control, the same membrane was hybridized in the same way with a β-actin specific sequence as a probe. When the N-terminal fragment of TIRAP cDNA was used as a probe, TIRAP transcripts from the knockout mice were detected at almost the same size as the band of wild-type mice. When the C-terminal fragment was used as a probe, the band detected in wild-type mice was not detected in knockout mice (Fig. 2, right).

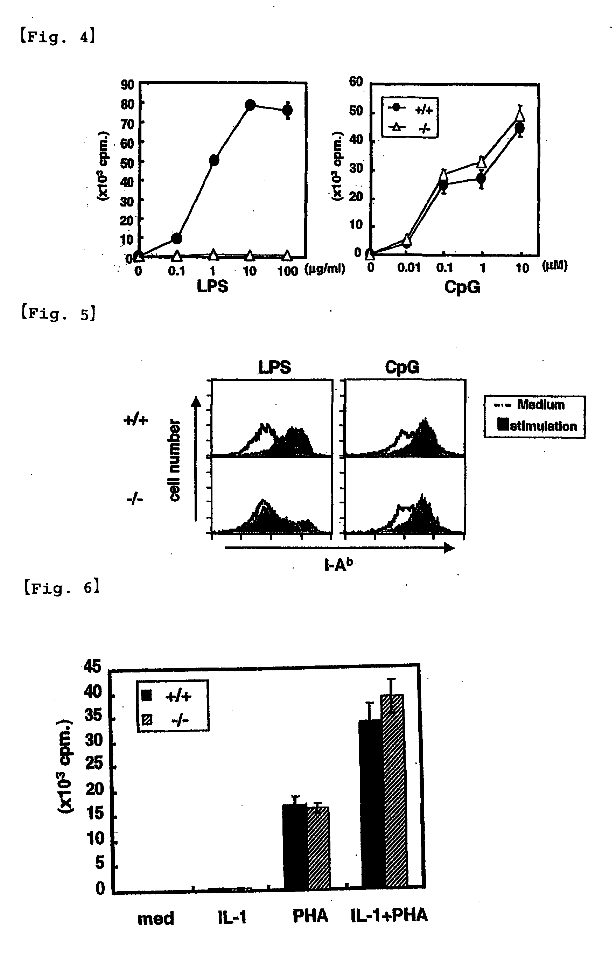

Furthermore, RT-PCR analysis indicated that the TIRAP genes in the knockout mice contained the neo genes. Moreover, stop codon appeared at the N-terminal portion of TIRAP (Fig. 3). When TIRAP genes are disrupted, TIRAP protein is not produced in knockout mice. TIRAP mice obtained in this way were healthy and showed no abnormal composition of B and T lymphocytes as determined by flow cytometry. Such TIRAP-knockout mice and its littermates were used for the examples.

### Example 2 (LPS responsiveness of splenocytes from TIRAP-knockout mice)

TIRAP is identified as an adaptor that specifically associates with TLR4, which is essential for recognition of LPS. Therefore, the responsiveness of splenocytes from wild-type and TIRAP^{-/-} mice responsive to LPS being TLR4 ligands was examined using LPS of Salmonella Minnesota Re-595 (Sigma). Furthermore, as control, responsiveness of splenocytes was examined using CpG oligodeoxynucleotides (CpG ODN) being TLR9 ligands. LPS and CpG ODN were prepared as described previously (Hemmi et al., Nature 408, 740-745, 2000) and used. Splenocytes (1 × 10⁵) from wild-type and TIRAP^{-/-} mice were isolated respectively, stimulated with LPS at various concentrations (0, 0.1, 1, 10 or 100 µg/ml) or with CpG ODN at various concentrations (0, 0.01, 0.1, 1 or 10 µg/ml), and cultured. After 48 hr of culture, 1 µCi of [³H]-thymidine (DuPont) was supplemented, and further cultured for 12 hr, and measured the uptake of [³H] by a β-scintillation counter (Packard). Wild-type splenocytes showed a dose-dependent proliferation to stimulation by LPS and CpG ODN. However, TIRAP^{-/-} splenocytes did not show any proliferative response to LPS stimulation, although they showed normal response to CpG ODN stimulation (Fig. 4).

Next, the expression of major histocompatibility complex (MHC) class II (I-A^{b}) on the surface of respective splenic B cells from wild-type and TIRAP^{-/-} mice in the response to Re-595 LPS and CpG ODN using 1 µg/ml of Re-595 LPS or 0.1 µM of CpG ODN, by flow cytometry. Respective splenic B cells (1 × 10⁶) from wild-type mice and TIRAP^{-/-} mice were cultured in the presence or absence of LPS or CpG ODN for 48 hr. After the culture, cells were harvested and stained by binding to I-A^{b} in the surface of these cells by using FITC-labeled antigens wherein Streptavidin labeled with Fluorescein isothiocyanate (FITC; Pharmingen) is bonded to biotin-conjugated anti-mouse I-A^{b} antibody (Pharmingen). Stained cells were analyzed on fluorescence-activated cell sorter Calibur (FACS Calibur) using Cell Quest software (Becton Dickinson). As a result, expression of MHC class II was enhanced on the surface of cells in wild-type splenic B cells when cultured with LPS or CpG ODN. In contrast, expression of MHC class II was severely impaired in TIRAP^{-/-} splenic B cells when cultured with LPS. However, expression of MHC class II was not impaired when cultured with CpG ODN (Fig. 5). Thus, TIRAP^{-/-} splenic B cells shows defective response to LSP.

### Example 3 (IL-1, PHA responsiveness of splenocytes from TIRAP-knockout mice)

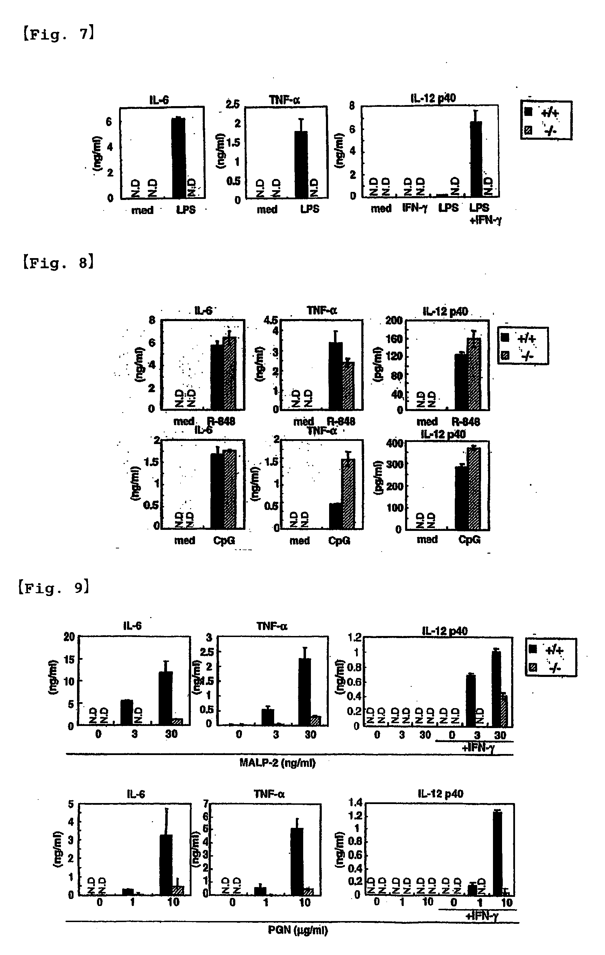

As shown in MyD88-, TRAF6-, and IRAK-4-deficient mice, signaling pathway via IL-1 receptor family utilizes the same signaling pathway as that of TLRs (Adachi et.al., Immunity. 9, 143-150, 1998; Lomaga et al., Gene Dev. 13, 1015-24, 1999; Naito et al., Gene Cells 4, 353-62, 1999; Suzuki et al., Nature 416, 750-56, 2002). To investigate the proliferation of splenocytes from TIRAP^{-/-} mice induced by IL-1 or Phytohemagglutinin (PHA) being co-stimulatory substance with IL-1, splenocytes from wild-type and TIRAP^{-/-} mice were cultured for 72 hr and proliferated in the presence of 100 U/ml of IL-1β, and of 10 ng/ml of PHA, and in the co-presence of IL-1 and PHA, then supplemented with 1 µCi of [³H]-thymidine, further cultured for 12 hr, and measured the uptake of [³H] with a β-scintillation counter. Proliferation of splenocytes was determined by measuring the [³H] level of [³H] thymidine ingested into a cell. Wild-type and TIRAP^{-/-} splenocytes displayed enhanced proliferation when cultured in the presence of IL-1, and of PHA, and in the co-presence of IL-1 and of PHA (Fig. 6). This demonstrates that TIRAP^{-/-} mice show a normal response to IL-1 or PHA.

### Example 4 (Inflammatory cytokine production of peritoneal macrophages from TIRAP-knockout mice in response to various TLR ligands)

Next, LPS-induced inflammatory cytokine production of peritoneal macrophages from wild-type and TIRAP^{-/-} mice was analyzed. 2 ml of 4% thioglycollate medium (DIFCO) were intraperitoneally injected into TIRAP^{-/-} and wild-type mice, respectively. Three days later, peritoneal exudate cells were isolated from peritoneal cavity of each mouse. The cells were cultured for 2 hr at 37°C in RPMI 1640 medium (GIBCO) supplemented with 10% fetal bovine serum (GIBCO), nonadherent cells were removed by washing with Hank's buffered salt solution: HBSS; GIBCO) at ice temperature, and adherent cells were used as peritoneal macrophages. Each obtained peritoneal macrophage was cultured with 1 ng/ml of Re-595 LPS for 24 hr, and concentration of IL-6, TNF-α and IL-12 in the culture supernatant was measured according to a common procedure by ELISA (Fig.7; means ± SD of triplicates, N.D. Not detected). Wild-type peritoneal macrophages produced IL-6, TNF-α and IL-12 in response to LPS. However, LPS-induced production of IL-6, TNF-α and IL-12 was not observed at all in TIRAP^{-/-} macrophages.

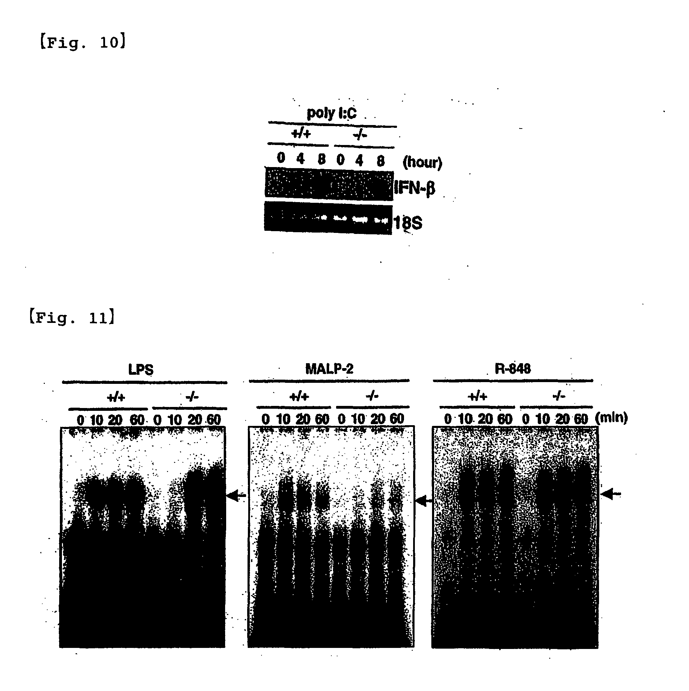

Similarly, to analyze the response to other TLR ligands, concentration of IL-6, TNF-α and IL-12 in the culture supernatant was measured with 10 nM of synthetic R-848 (TLR7 ligand; synthesized and purified as described previously (Hemmi et al., Nature Immunol. 3, 196-200, 2002)) and 1 µM of CpG ODN (TLR9 ligand) by ELISA (Fig. 8). Wild-type and TIRAP^{-/-} macrophages produced similar level of TNF-α, IL-6 and IL-12 in response to CpG ODN and R-848. IL-6, TNF-α and IL-12 in the culture supernatant were measured using mycoplasmal lipopeptide MALP-2 being TLR2 ligand (synthesized and purified as described in Takeuchi-MALP) and Staphylococcus aureus peptidoglycan (PGN) (Fluka) at various concentrations (Fig. 9). Wild-type macrophages showed a dose-dependent production of TNF-α, IL-6 and IL-12 in response to MALP-2. However, production of TNF-α, IL-6, and IL-12 was severely impaired in TIRAP^{-/-} mice. PGN-induced expression of TNF-α, IL-6, and IL-12 was also significantly reduced. Next, peritoneal macrophages of wild-type and TIRAP^{-/-} mice were stimulated with Poly I:C being TLR3 ligand (Amersham) for 4 hr and 8 hr. Total RNA was extracted, and the expression of IFN-β mRNA was analyzed by Northern blot method. At that time, 18S ribosomal RNA was used as control. Expression of IFN-β mRNA was induced in both wild-type and TIRAP^{-/-} mice (Fig. 10).

These results demonstrated that TIRAP^{-/-} mice showed a normal response to microbial components recognized by TLR3, TLR7, and TLR9 like wild-type mice, but the response to TLR2 and TLR4 ligands was impaired.

### Example 5 (Activation of signaling cascades of peritoneal macrophages of TIRAP-knockout mice in response to various TLR ligands)

Furthermore, activation of NF-κB in response to LPS, MALP-2 and R-848 was examined. Nuclear extracts were prepared according to phenol-chloroform-petroleum ether extraction procedure as described previously (Takeuchi-MALP, Hemmmi 2002), and peritoneal macrophages(1 × 10⁶) were stimulated with 100 ng/ml of Re-595 LPS (Sigma), 3 ng/ml of MALP-2, and 10 nM of R-848 for 0, 10, 20, and 60 min, respectively to purify the nuclear extracts from the cells, incubated with a specific probe for NF-□B DNA binding site, electrophoresed, visualized by autoradiography according to the method described previously (Kawai et al., Immunity 11: 115-22; 1999), and determined by EMSA (Fig. 11; Arrows indicate induced NF-κB complex). NF-κB DNA binding effect in response to LPS stimulation was observed in nuclear extracts of wild-type macrophages at 10 min. However, it was not observed in nuclear extracts of TIRAP^{-/-} macrophages at 10 min, while significant amount was observed after 20 min. NF-κB DNA binding effect in response to MALP-2 stimulation was observed in nuclear extracts of wild-type macrophages at 10 min, but significantly inhibited in TIRAP^{-/-} mice. Moreover, NF-κB DNA binding effect in response to R-848 stimulation was normally observed in both wild-type and TIRAP^{-/-} mice.

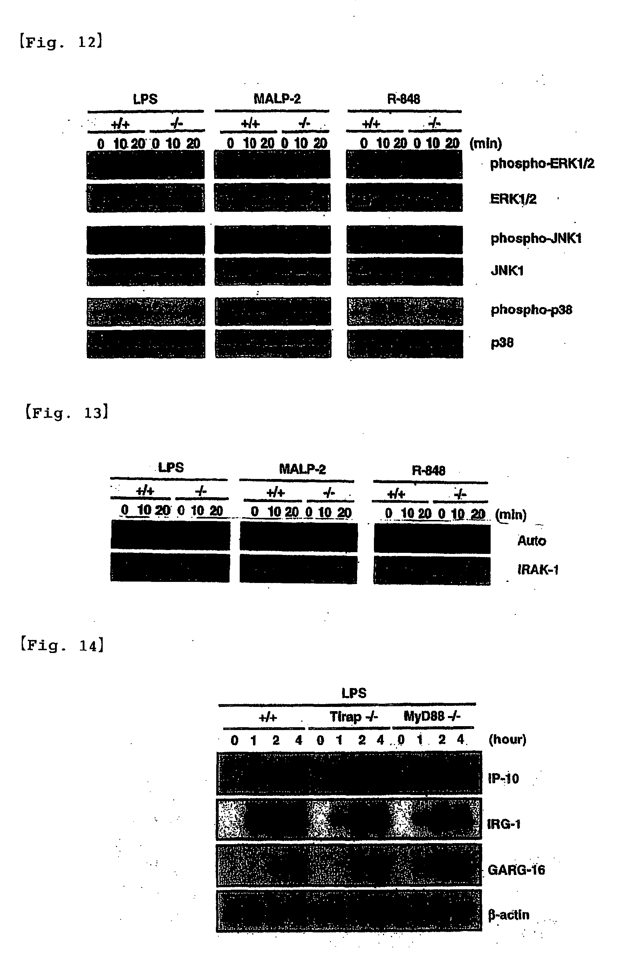

Next, peritoneal macrophages from wild-type and TIRAP^{-/-} mice were stimulated with 100 ng/ml of LPS, 3 ng/ml of MALP-2, and 100 nM of R848 for 10 min and 20 min, respectively. The cells were then lysed in the lysis buffer containing 1.0% Nonidet P-40, 150 mM NaCl, 20 mM tris-C1(pH 7.5), 5 mM EDTA and protease inhibitor cocktail (Roche). The cell lysates were dissolved by SDS-PAGE and transferred onto a PVDF membrane (BioRad). The above-mentioned PVDF membrane was blotted with antigens specific to ERK1/2, JNK, and p38 (Santa Cruz), and antigens specific to phosphorylated ERK 1/2, phosphorylated JNK, and phosphorylated p38 (Cell Signaling) and Western blot analysis was conducted by visualizing with an enhanced chemiluminescence system (NEN Life Science Product) (Fig. 12). By respective stimulation of LPS, MALP-2 and R-848, ERK1/2, JNK and p38 were phosphorylated and activation was induced in wild-type macrophages. LPS-induced activation was observed in peritoneal macrophages from TIRAP^{-/-} mice though there was a time lag. The result is quite similar to those observed in MyD88^{-/-} mice (Kawai et al.: Immunity 11: 115-22; 1999). Moreover, MALP-2-induced activation of ERK1/2, JNK, and p38 was not observed, but R-848-induced activation was the similar level of wild-type macrophages, indicating that TIRAP is critically involved in TLR2-mediated signaling pathway.

IRAK-1 is activated in the downstream of MyD88. To address whether TIRAP functions in the upstream of IRAK-1, lysates of peritoneal macrophages stimulated with LPS, MALP-2 or R-848 were immunoprecipitated using rabbit anti-IRAK-1 polyclonal antibody (Hayashibara Biochemical Laboratories). In vitro kinase assay was conducted as described previously (Sato et al. II), and kinase activation of IRAK-1 was measured. The same lysates were blotted with anti-IRAK-1 antibody, and the effect of IRAK-1 was analyzed (Fig. 13). As a result, in wild-type macrophages, stimulation with LPS, MALP-2, and R-848 induced autophosphorylation (Auto) of IRAK-1. Although in TIRAP^{-/-} mice autophosphorylation was induced in response to R-848, autophosphorylation in response to LPS and MALP-2 was not observed. These results indicate that TIRAP acts at a proximal level of IRAK similar to MyD88, but unlike MyD88, TIRAP is specifically involved in TLR2- and TLR4-mediated activation of IRAK.

### Example 6 (LPS-induced activation of MyD88-independent signaling pathway in peritoneal macrophages from TIRAP-knockout mice)

Next, to address whether TIRAP deficiency affects the MyD88-independent signaling, peritoneal macrophages from wild-type, TIRAP^{-/-} and MyD88^{-/-} mice were stimulated with 100 ng/ml of Re-595 LPS for 0, 1, 2, and 4 hr, respectively, total RNA (5 µg) was extracted, and the expression of IP-10, GARG-16, and IRG-1 being IFN-inducible genes was analyzed by Northern blot method respectively. Moreover, the same membrane was rehybridized with a β-actin specific probe, and mRNA induction was analyzed (Fig. 14). LPS-induced expression of IP-10, GARG-16, and IRG-1 was similarly observed in wild-type, TIRAP^{-/-} and MyD88^{-/-} peritoneal macrophages.

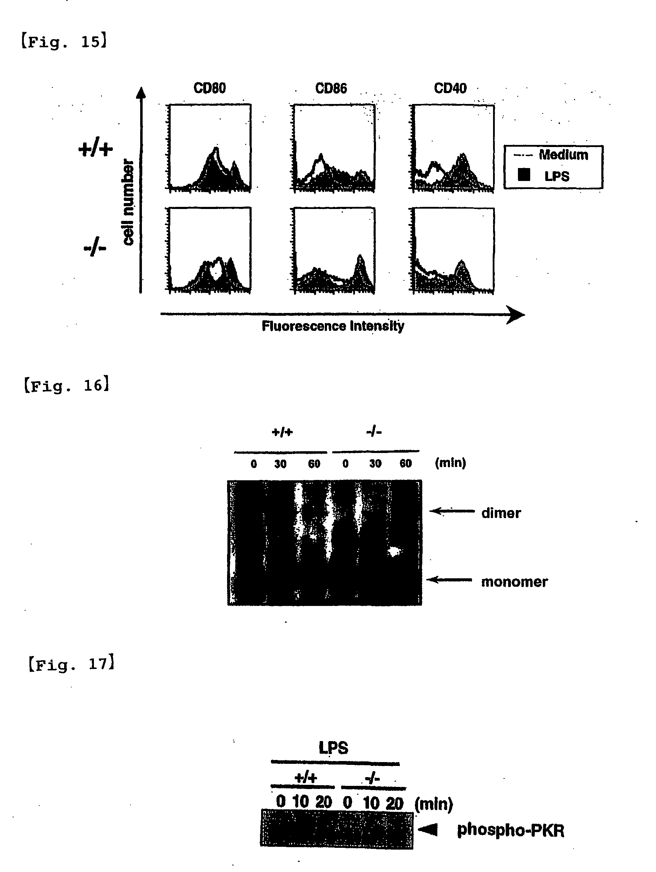

Bone marrow cells from wild-type and TIRAP^{-/-} mice were cultured for 6 days using 10 ng/ml of GM-CSF and immature dendritic cells were harvested. They were further cultured in new medium of 0.1 ng/ml in the presence or absence of Re-595 LPS for 2 days. Next, CD40, CD80, and CD86 were reacted with biotin conjugated antibody, then stained with PE-labeled Streptavidin. Strained cells were analyzed with fluorescence-activated cell sorter Calibur (FACS Calibur) using Cell Quest software (Becton Dickinson) (Fig. 15). As a result, in wild-type mice, enhanced expression of MHC class II and CD80, CD86, and CD40 being co-stimulatory molecules thereof was induced. Furthermore, in TIRAP^{-/-}, the expression of MHC class II, CD80, CD86, and CD40 were similarly enhanced in response to LPS.

In the MyD88-independent signaling, IRF-3 has been known to be activated in response to LPS (Kawai et al.: J Immunol. 167, 5887-94, 2001). Therefore, to analyze LPS-induced activation of IRF-3, peritoneal macrophages were stimulated in 100 ng/ml of LPS for 1 or 2 hr, cell lysates were prepared, subjected to native polyacrylamide gel electrophorisis, immunoblotted with anti-IRF-3 antibody, and monomeric and dimeric forms of IRF-3 were detected (Fig. 16). Peritoneal macrophages from wild-type mice formed homodimers. It was similarly observed in peritoneal macrophages from TIRAP^{-/-} mice, indicating LPS-induced activation of IRF-3 was not affected in the absence of TIRAP. Moreover, peritoneal macrophages were stimulated with LPS for 10 or 20 min, cell lysates were prepared, and phospho-PKR was detected by Western blot analysis using anti-phosphorylated PKR antibody (Biosource) (Fig. 17). In peritoneal macrophages from wild-type mice, phosphorylation of PKR was observed by LPS stimulation for 10 min, whereas in peritoneal macrophages from TIRAP^{-/-} mice, phosphorylation of PKR was observed by LPS stimulation for 20 min, suggesting that TIRAP is not essential for the LPS-induced activation of the MyD88-independent signaling pathway.

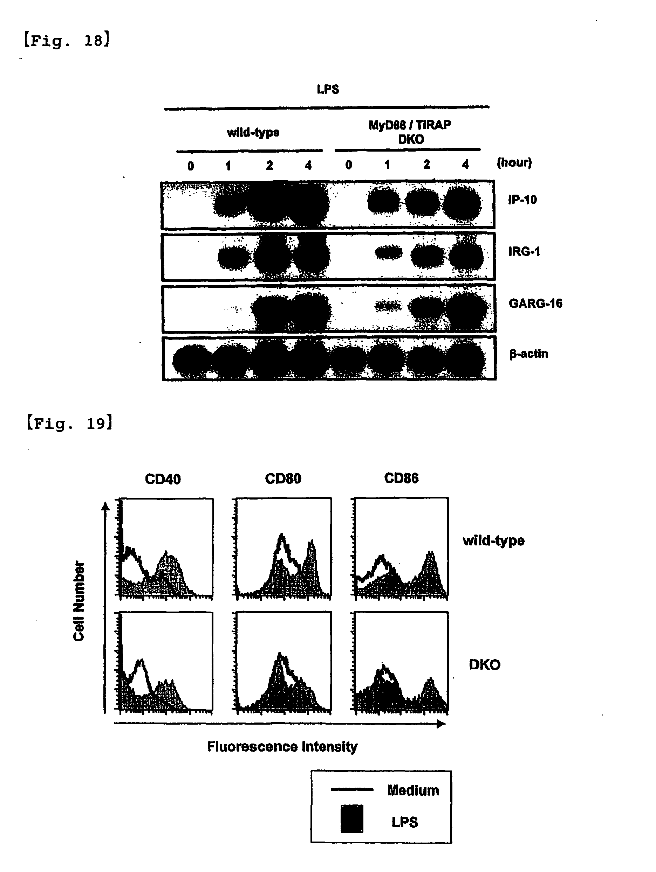

Thus, LPS-induced activation of MyD88-independent signaling pathway was not impaired in TIRAP^{-/-} mice. In other words, there remains some possibilities that MyD88 compensates for a deficiency of TIRAP to activate MyD88-independent signaling pathway. Therefore, double-knockout mice wherein both TIRAP and MyD88 genes are deleted, were generated, and LPS-induced expression of IFN-inducible genes was analyzed. When peritoneal macrophages from TIRAP/MyD88 double-knockout mice were stimulated with LPS, mRNA expression of IP-10, GARG-16, and IRG-1 was induced in a similar level to wild-type peritoneal macrophages (Fig. 18). Furthermore, in dendritic cells from TIRAP/MyD88 double-knockout mice, the expression of MHC class II as well as CD80, CD86, and CD40 was enhanced in response to LPS just like wild type cells (Fig. 19). These results were similar to those observed in TIRAP^{-/-} and MyD88^{-/-} mice. In other words, it is shown that TIRAP acts in the TLR4-mediated MyD88-dependent, but not MyD88-independent, signaling pathway in response to LPS, and also is critically involved in the TLR2-mediated signaling pathway. Previous studies have demonstrated that MyD88 is essential for the inflammatory response via any TLR family members, but it was found that TIRAP provides a specificity for both TLR2- and TLR4-mediated signaling pathways.

### Industrial applicability

As the non-human animal models non-responsive to endotoxins and lipoproteins/lipopeptides such as TIRAP-knockout mice of the present invention are non-responsive to ligands recognized by TRL2 such as lipoproteins/lipopeptides and ligands recognized by TLR4 such as endotoxins, it would be possible to screen substances promoting or suppressing response to ligands recognized by TLR2 and TLR4, and therefore to obtain new useful information to elucidate the molecule structure of establishment of bacterial infections. They can be useful models to elucidate the effect mechanism of ligands recognized by TLR2 and TLR4, to develop therapeutic agents for various infections, and to construct a treatment strategy for endotoxin shock, or for bacterial infections such as tuberculosis and pneumonia.

## Claims

1. A non-human animal model non-responsive to endotoxins and lipoproteins/lipopeptides, wherein the function of the TIRAP gene is deleted in its chromosome and the responsiveness to ligands recognized by TLR2 and TLR4 is specifically impaired.

2. The non-human animal model non-responsive to endotoxins and lipoproteins/lipopeptides according to claim 1, wherein the non-human animal model is non-responsive to LPS, PGN, and MALP-2, and responsive to CpG ODN, IL-1, Poly I:C and R-848.

3. The non-human animal model non-responsive to endotoxins and lipoproteins/lipopeptides according to claim 1 or 2, wherein the non-human animal is a mouse.

4. The non-human animal model non-responsive to endotoxins and lipoproteins/lipopeptides according to claim 3, wherein the mouse is obtained by a process comprising steps of: obtaining a TIRAP gene by screening a murine genomic library using a probe for the mouse TIRAP gene; constructing a targeting vector by replacing a whole or a part of the coding sequences of the TIRAP gene with a marker gene; linearizing the targeting vector and injecting it into embryonic stem cells; microinjecting the targeted stem cells wherein the function of TIRAP gene is deleted, into the mouse blastocysts to generate chimeric mice; breeding the chimeric mice and wild-type mice to generate heterozygous mice; and intercrossing the heterozygous mice.

5. A method for screening substances promoting or suppressing the response to ligands recognized by TLR2 or TLR4, or to the inclusion thereof, wherein the response to ligands recognized by TLR2 or TLR4, or to the inclusion thereof in the immunocytes derived from the non-human animal according to any one of claims 1 to 4 is measured/estimated, by using the immunocutes, a test substance, and ligands recognized by TLR2 or TLR4, or the inclusion thereof.

6. The method for screening substances promoting or suppressing the response to ligands recognized by TLR2 or TLR4, or to the inclusion thereof, wherein the response to ligands recognized by TLR2 or TLR4, or to the inclusion thereof in the non-human animal according to any one of claims 1 to 4 is measured/estimated, by using the non-human animal, a test substance and ligands recognized by TLR2 or TLR4, or the inclusion thereof.

7. A substance promoting or suppressing the response to ligands recognized by TLR2 or TLR4, or to the inclusion thereof, obtained by the method for screening a substance promoting or suppressing the response to ligands recognized by TLR2 or TLR4, or to the inclusion thereof according to claim 5 or 6.
